(19) Europäisches Patentamt
European Patent Office
Office européen des brevets

(11) Veröffentlichungsnummer: **0 313 924 B1**

(12) **EUROPÄISCHE PATENTSCHRIFT**

(45) Veröffentlichungstag der Patentschrift: **03.06.92**

(51) Int. Cl.5: **C07C 237/04**, C07C 231/00

(21) Anmeldenummer: **88116977.5**

(22) Anmeldetag: **13.10.88**

(54) **Verfahren zur Herstellung von N-Acetylphenylalanin.**

(30) Priorität: **30.10.87 DE 3736861**

(43) Veröffentlichungstag der Anmeldung:
**03.05.89 Patentblatt 89/18**

(45) Bekanntmachung des Hinweises auf die Patenterteilung:
**03.06.92 Patentblatt 92/23**

(84) Benannte Vertragsstaaten:
**BE CH DE FR GB IT LI NL**

(56) Entgegenhaltungen:

**SYNTHESIS, Nr. 12, Dezember 1983, Seiten 1041-1043, Stuttgart, DE; J.M. ROPER et al.: "Synthesis of phenylalanines in high enantiomeric excess via enzymatic resolution"**

**"Organic syntheses", "collective volume 2" 1943, Seiten 1-3, John Wiley & Sons, Inc., New York, US**

**"Organic syntheses", "collective volume 2", 1943, Seiten 491-493, John Wiley & Sons, Inc., New York, US**

**CHEMICAL ABSTRACTS, Band 104, Teil 9, 3. März 1986, Seite 659, Zusammenfassung Nr. 68622a, Columbus, Ohio, US; & JP-A-60 185 752**

**CHEMICAL ABSTRACTS, Band 104, Teil 21, 26. Mai 1986, Seite 677, Zusammenfassung Nr. 186850w, Columbus, Ohio, US; & JP-A-60 199 864**

(73) Patentinhaber: **HOECHST AKTIENGESELLSCHAFT**
**Postfach 80 03 20**
**W-6230 Frankfurt am Main 80(DE)**

(72) Erfinder: **Erpenbach, Heinz, Dr.**
**Oberbuschweg 22**
**W-5000 Köln(DE)**
Erfinder: **Gehrmann, Klaus, Dr.**
**Geschwister-Scholl-Strasse 32**
**W-5042 Erftstadt(DE)**
Erfinder: **Hörstermann, Peter, Dr.**
**Theodor-Heuss-Strasse 2**
**W-5042 Erftstadt(DE)**
Erfinder: **Jägers, Erhard, Dr.**
**Hemberger Strasse 75**
**W-5303 Bornheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Erfindung betrifft ein Verfahren zur Herstellung von N-Acetylphenylalanin durch Ringöffnung von 2-Methyl-4-benzyliden-1,3-oxazolin-5-on mit Wasser zu 2-Acetaminozimtsäure und deren anschließende katalytische Hydrierung.

2-Methyl-4-benzyliden-1,3-oxazolin-5-on ist seinerseits aus Benzaldehyd und N-Acetylglycin erhältlich. Die Reaktionen werden durch folgende Formelschemata veranschaulicht:

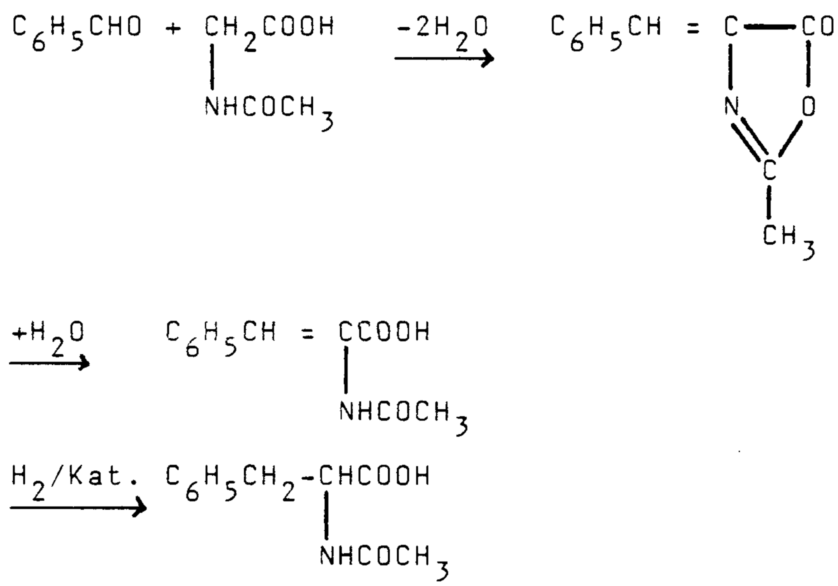


(vergl. Organic Synthesis, Coll. Vol. 2 (1943), Seiten 1 bis 3 und 491 bis 493).

Ringöffnung und katalytische Hydrierung erfordern das Arbeiten in einem organischen Lösemittel. Für die Ringöffnung werden Aceton/Wasser-Gemische, aber auch mit Wasser mischbare Ether wie Tetrahydrofuran, Dioxan und Ethylenglykol-dimethylether genannt. Für die katalytische Hydrierung haben sich vor allem Alkohol/Wasser-Gemische bewährt.

Für das Verfahren zur Herstellung von N-Acetylphenylalanin wäre es eine erhebliche Vereinfachung, wenn sowohl Ringöffnung als auch Hydrierung in ein und demselben Lösemittel durchgeführt werden könnten.

Für die Ringöffnung mit Wasser muß das organische Lösemittel mit Wasser mischbar sein. Alkohole sind ungeeignet, da sie mit dem 2-Methyl-4-benzyliden-1,3-oxazolin-5-on-Ring unter Bildung von Estern reagieren können (vergl. Synthesis, Dez. 1983, Seiten 1041 bis 1043). Andererseits erscheint eine katalytische Hydrierung in Aceton oder Aceton/Wasser-Gemischen undurchführbar, da die Möglichkeit einer Hydrierung des Acetons besteht (Houben-Weyl, Methoden der Organischen Chemie, Band 4, Teil 1C, (1980), Seite 192 ff).

Überraschenderweise wurde jedoch gefunden, daß in einem bestimmten Druck- und Temperaturbereich unter milden Bedingungen und in Gegenwart eines bestimmten Katalysators eine Hydrierung der Doppelbindung in der 2-Acetaminozimtsäure zu Acetylphenylalanin auch in Aceton/Wasser-Gemischen möglich ist, ohne daß es zu einer Hydrierung des Acetons kommt. Auf diese Weise lassen sich Ringöffnung und katalytische Hydrierung ohne Wechsel des Lösemittels durchführen, was nicht nur eine verfahrensmäßige Vereinfachung, sondern auch eine deutliche Ausbeuteerhöhung bedeutet, da auf eine Zwischenisolierung der N-Acetaminozimtsäure verzichtet werden kann.

Im einzelnen ist das Verfahren der Erfindung nunmehr dadurch gekennzeichnet, daß beide Reaktionsstufen in einem Gemisch aus einem aliphatischen $C_3$- bis $C_{10}$-Keton oder einem mit Wasser mischbaren Ether und Wasser als Lösemittel durchgeführt werden und die Hydrierung der 2-Acetaminozimtsäure bei Temperaturen von 10 bis 50 °C und Drucken von 1 bis 15 bar in Gegenwart eines Palladium-Trägerkatalysators stattfindet.

Das Verfahren der Erfindung kann weiterhin bevorzugt und wahlweise dadurch gekennzeichnet sein, daß

a) man als Lösemittel Gemische aus Aceton und Wasser einsetzt;

b) man als Lösemittel Gemische aus Tetrahydrofuran, Dioxan oder Ethylenglykoldimethylether und Wasser einsetzt.

c) das Lösemittelgemisch 5 bis 50 Gewichts% Wasser enthält.

Dabei geht man im allgemeinen so vor, daß 2-Methyl-4-benzyliden-1,3-oxazolin-5-on durch Erwärmen in Wasser/Aceton-Gemischen zu 2-Acetaminozimtsäure gespalten und die Reaktionslösung anschließend mit dem Palladium-Trägerkatalysator versetzt und mit Wasserstoff hydriert wird. Das aus der Reaktionslösung gewonnene N-Acetylphenylalanin dient beispielsweise zur Herstellung von Phenylalanin.

Der Palladium-Trägerkatalysator kann 0,5 bis 10 Gewichts% Palladium enthalten. Geeignete Träger sind z. B. Kieselgel, Aktivkohle, Aluminiumoxid und Bariumsulfat. Die katalytische Hydrierung erfolgt bevorzugt in einem Druckbereich von 1 bis 10 bar und bei Temperaturen von 15 bis 50 °C. Die einzusetzende Menge Trägerkatalysator kann in weiten Bereichen variieren, doch ist schon aus Kostengründen eine geringe Menge vorzuziehen. Als Lösemittel für beide Reaktionsstufen sind Keton/Wasser-Gemische, insbesondere Aceton/Wasser-Gemische, wegen der besseren Handhabbarkeit und auch aus Preisgründen gegenüber mit Wasser mischbaren Ethern bevorzugt.

Beispiel

6 kg 2-Methyl-4-benzyliden-1,3-oxazolin-5-on werden in 38 kg Aceton gelöst und auf Rückflußtemperatur erwärmt. Anschließend werden 32 kg Wasser hinzugegeben und das Gemisch 3 Stunden unter Rückfluß erhitzt. Man kühlt den Ansatz ab, fügt 2,69 kg eines Pd-Träger-Katalysators (Träger: Kieselgel; 1,5 Gewichts% Pd) zu, spült mit Stickstoff, versetzt mit Wasserstoff und hydriert bei 30 °C und 2 bar Druck. Nach Aufnahme der theoretischen Wasserstoffmenge wird abgekühlt, der Katalysator abfiltriert und das Lösemittelgemisch entfernt. Die Ausbeute an N-Acetylphenylalanin beträgt 95 % d. Th.

## Patentansprüche

**1.** Verfahren zur Herstellung von N-Acetylphenylalanin durch Ringöffnung von 2-Methyl-4-benzyliden-1,3-oxazolin-5-on mit Wasser zu 2-Acetaminozimtsäure und deren anschließende katalytische Hydrierung, dadurch gekennzeichnet, daß beide Reaktionsstufen in einem Gemisch aus einem aliphatischen $C_3$- bis $C_{10}$-Keton oder einem mit Wasser mischbaren Ether und Wasser als Lösemittel durchgeführt werden und die Hydrierung der 2-Acetaminozimtsäure bei Temperaturen von 10 bis 50 °C und Drucken von 1 bis 15 bar in Gegenwart eines Palladium-Trägerkatalysators stattfindet.

**2.** Verfahren nach Anspruch 1, dadurch gekennzeichnet, daß man als Lösemittel Gemische aus Aceton und Wasser einsetzt.

**3.** Verfahren nach Anspruch 1, dadurch gekennzeichnet, daß man als Lösemittel Gemische aus Tetrahydrofuran, Dioxan oder Ethylenglykoldimethylether und Wasser einsetzt.

**4.** Verfahren nach einem der vorhergehenden Ansprüche, dadurch gekennzeichnet, daß das Lösemittelgemisch 5 bis 50 Gewichts% Wasser enthält.

## Claims

**1.** A process for the preparation of N-acetylphenylalanine by opening the ring of 2-methyl-4-benzylidene-1,3-oxazolin-5-one with water to give 2-acetaminocinnamic acid and subsequently catalytically hydrogenating the latter, which comprises carrying out the two reaction stages in a mixture of an aliphatic $C_3$-ketone to $C_{10}$-ketone or a water-miscible ether and water as the solvent, and carrying out the hydrogenation of the 2-acetaminocinnamic acid at temperatures of 10 to 50 °C and pressures of 1 to 15 bar in the presence of a supported palladium catalyst.

**2.** The process as claimed in claim 1, wherein mixtures of acetone and water are employed as the solvent.

**3.** The process as claimed in claim 1, wherein mixtures of tetrahydrofuran, dioxane or ethylene glycol dimethyl ether and water are employed as the solvent.

**4.** The process as claimed in one of the preceding claims, wherein the solvent mixture contains 5 to 50%

by weight of water.

**Revendications**

1. Procédé pour la fabrication de N-acétylphénylalanine par ouverture de cycle de la 2-méthyl-4-benzylidène-1,3-oxazoline-5-one par l'eau en acide 2-acétamido-cinnamique suivie de son hydrogénation catalytique, caractérisé en ce que les deux étapes réactionnelles sont mises en oeuvre dans un mélange d'une cétone aliphatique en $C_3$-$C_{10}$ ou d'un éther miscible avec l'eau et d'eau comme solvant et l'hydrogénation de l'acide 2-acétamido-cinnamique a lieu à des températures de 10 à 50 °C et sous des pressions de 1 à 15 bar en présence d'un catalyseur au palladium sur support.

2. Procédé selon la revendication 1, caractérisé en ce que l'on utilise comme solvants des mélanges d'acétone et d'eau.

3. Procédé selon la revendication 1, caractérisé en ce que l'on utilise comme solvants des mélanges de tétrahydrofuranne, dioxanne ou éther diméthylique d'éthylèneglycol et d'eau.

4. Procédé selon l'une quelconque des revendications précédentes, caractérisé en ce que le mélange solvant contient 5 à 50% en poids d'eau.